# EUROPEAN PATENT APPLICATION

(11) **EP 1 291 030 A1**
(43) Date of publication of application: **12.03.2003**
(21) Application number: 01830576.3
(22) Date of filing: 10.09.2001
(51) Int. Cl.: A61M 5/32

(54) **Safety accessory for a hypodermic syringe**

(71) Applicant: Restelli, Sergio, 00100 Rome (IT)
(72) Inventor: Restelli, Sergio, 00100 Rome (IT)
(74) Representative: Petruzziello, Aldo

(57) **Abstract**

In a hypodermic syringe (100) comprising a syringe body (1) hollow inside and open at the front and at the rear, a plunger slidable inside the syringe body (1) with an injection stroke extending from a retracted syringe-filling position to a forward syringe-emptying position, said plunger being provided at the rear with a shaft (5) that can be operated manually and brought out of the syringe body through the rear end thereof, and an injection needle (10) integral with a needle-carrier (11) attachable to the front end (2) of the syringe body (1), a safety accessory (50) is provided comprising a supporting element (20) removably mounted on the front part of the syringe body (1) and able to receive the needle-carrier (11) and an operating element (30) slidably mounted on the syringe body and comprising abutment means (39, 40) able to cooperate with the supporting element (20) to cause disengagement of the supporting element (20) from the front part of the syringe body, so that the supporting element (20) integral with the needle carrier (11) is retained inside the operating element (30), to leave the needle (10) protected inside the operating element (30), avoiding accidental needle sticks.

## Description

The present invention refers to a safety accessory for a hypodermic syringe.

As is known, a syringe generally comprises a cylindrical body open at the rear to accommodate a plunger. An internally hollow needle is mounted at the head of the syringe body. By retracting the plunger the liquid contained in a vial is drawn into the syringe body through the needle. By pressing on the plunger the liquid contained inside the syringe body is injected, through the needle, into the patient's body.

Syringes generally present drawbacks from a safety standpoint concerning the operations for assembly and removal of the needle. Furthermore, once the syringe has been used, the needle remains exposed at the top of the syringe body, with the risk of accidental injuries or needle sticks.

This drawback is overcome in part by European patent EP 0636381 which discloses a protective device for syringe needles. In this case, when the syringe plunger reaches the end of its stroke, the front end of the plunger shaft catches the needle. When the injection is completed the user must manually retract the shaft; in this manner the needle is pulled by the head of the shaft inside the syringe body into a safety position which avoids accidental needle sticks.

Said solution presents problems during hooking of the needle carrier and has the drawback that the user, having completed the injection, can forget to carry out retraction of the shaft, leaving the needle exposed and thus rendering the protective device ineffective.

Patent application PCT WO 99/37345 discloses a disposable safety syringe which provides a needle-covering sleeve axially mounted on the syringe body and slidable from a retracted position, in which it leaves the needle exposed to allow injection, to a forward position in which it completely covers the needle, preventing re-use of the syringe and acting as a protection against accidental needle sticks.

Once the injection has been carried out the sleeve is automatically brought into the extended safety position, by means of an automatic mechanism and without any intervention by the user. However, said solution presents a certain complexity for provision and movement of the needle-covering sleeve.

The object of the present invention is to eliminate the drawbacks of the prior art, providing a safety accessory for a hypodermic syringe that is cheap and simple to make.

Another object of the present invention is to provide such a safety accessory that is practical for the user and versatile, so that it can be fitted to different types of hypodermic syringes.

Yet another object of the present invention is to provide such a safety accessory for a hypodermic syringe that is extremely safe and able to prevent accidental injuries both during assembly/removal of the needle and after use of the syringe.

These objects are achieved in accordance with the invention with the characteristics listed in appended independent claim 1.

Advantageous embodiments of the invention are apparent from the dependent claims.

The disposable syringe according to the invention comprises a syringe body hollow on the inside and open at the front and rear, a plunger slidable inside the syringe body with an injection stroke extending from a retracted syringe-filling position to an extended syringe-emptying position, and an injection needle integral with a needle-carrier attachable to the front end of the syringe body. The plunger is provided at the rear with a shaft that can be operated manually and brought out of the syringe body through the rear end thereof.

The peculiarity of the invention is represented by the fact that the safety accessory comprises a supporting element removably mounted on the front part of the syringe body and able to receive the needle-carrier, and an operating element slidably mounted on the syringe body and comprising abutment means able to cooperate with the supporting element to cause disengagement of the supporting element from the front part of the syringe body. In this manner the supporting element, integral with the needle-carrier, is retained inside the operating element, and the needle remains protected inside the operating element, avoiding accidental needle sticks.

The advantages of the safety accessory according to the invention are obvious. In fact, this accessory, besides being effective and safe in that it allows the needle to be removed in the utmost safety, is extremely simple from a structural and manufacturing standpoint, thus having low production costs. Further, the safety accessory is extremely versatile, in that it can be fitted on different types of syringe, without modifications of the syringe structure and is easily and directly usable by the user.

Further characteristics of the invention will be made clearer by the detailed description that follows, referring to purely exemplary and therefore non-limiting embodiments thereof, illustrated in the appended drawings in which:
Figure 1 is an exploded, axonometric view illustrating a hypodermic syringe and a safety accessory according to the invention;
Figure 2 is an axial sectional view of a first element of the safety accessory according to the invention;
Figure 3 is a side view of a second element of the safety accessory according to the invention;
Figure 4 is an axial sectional view of the second element of the safety accessory taken along the plane IV-IV of Figure 3;
Figure 5 is a cross sectional view of the second element of the safety accessory, along sectional plane V-V of Figure 4;
Figure 6 is a perspective view, illustrating the hypodermic syringe of Figure 1 assembled with the safety accessory;
Figure 7 is an enlarged axial sectional view, taken along the sectional plane VII-VII of Figure 6, in which for greater clarity the syringe body and the needle are not shown in section;
Figure 8 is a perspective view, illustrating the syringe of Figure 6 during operation of the safety accessory;
Figure 9 is an axial sectional view along sectional plane IX-IX of Figure 8 in which, for greater clarity, the syringe body and the needle are not shown in section;
Figure 10 is a perspective, exploded view illustrating the syringe of Figure 8, after use, in which the needle has been removed from the safety accessory according to the invention;
Figure 11 is an axial sectional view along sectional plane XI-XI of Figure 10, illustrating the safety accessory in a situation ready for disposal.

A safety accessory for a hypodermic syringe according to the invention will be described with the aid of the figures. The safety accessory is denoted by reference numeral 50 and the hypodermic syringe is denoted as a whole with reference numeral 100.

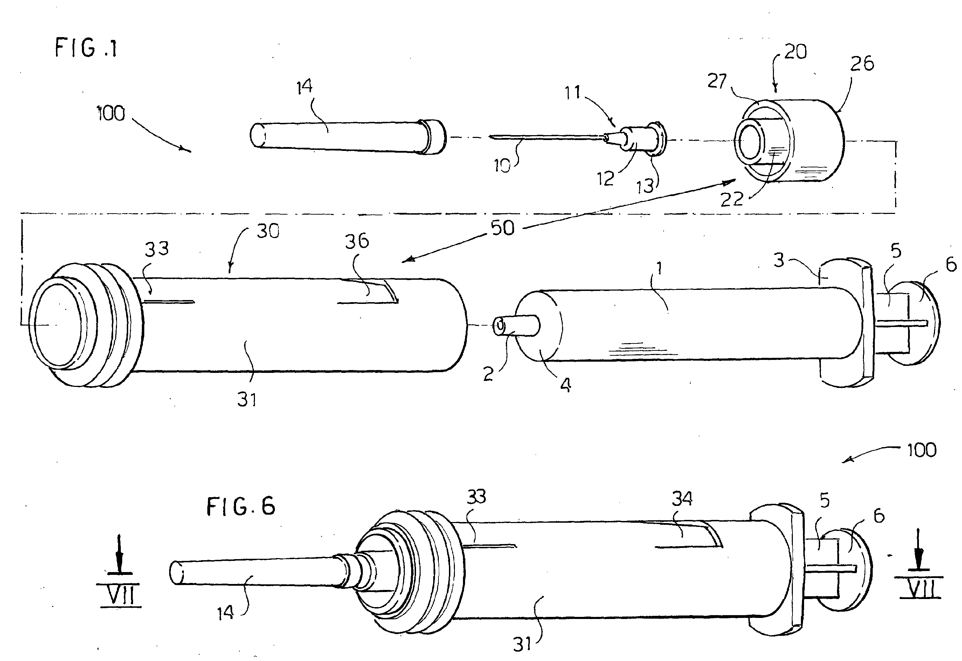

With reference for now to Figure 1 in particular, the syringe 100 comprises a cylindrical body 1, hollow on the inside, defining a cylindrical chamber. The rear end of the body 1 is open toward the outside and near its rear end the body 1 has a flange 3 which protrudes radially outward.

The front end of the body 1 has a tapered part 4 from which a head 2 protrudes axially. The head 2 is hollow on the inside, open toward the outside and is substantially shaped like a cylindrical tang with a smaller diameter than the diameter of the body 1.

A needle 10 is supported by a needle-carrier 11. The needle-carrier 11 comprises a cylindrical block 12 which has an axial hole to receive one end of the needle 10. In its free end the cylindrical block 12 of the needle carrier 11 has a thread 13 protruding radially from its outer side surface. Defined axially inside the cylindrical body 12 of the needle carrier is a substantially cylindrical or frusto conical seat, having a diameter substantially equal to the outside diameter of the front or head 2 of the syringe body. The needle 10 is covered by means of a needle guard 14 which snaps or screws onto the body 12 of the needle-carrier 11.

The syringe 100 comprises a plunger, per se known and therefore not illustrated in the figures, which is disposed inside the cylindrical body 1. The plunger is connected to the end of a shaft 5. The other end of the shaft 5 has an operating flange 6 disposed outside the syringe body 1 so as to be able to be operated by the user.

The safety accessory 50 comprises two elements. A first supporting element 20 to support the needle carrier 11 and a second operating element 30 which can be operated by the user to release the supporting element 20 from the head of the syringe body to cover the needle 10 in a position of maximum safety.

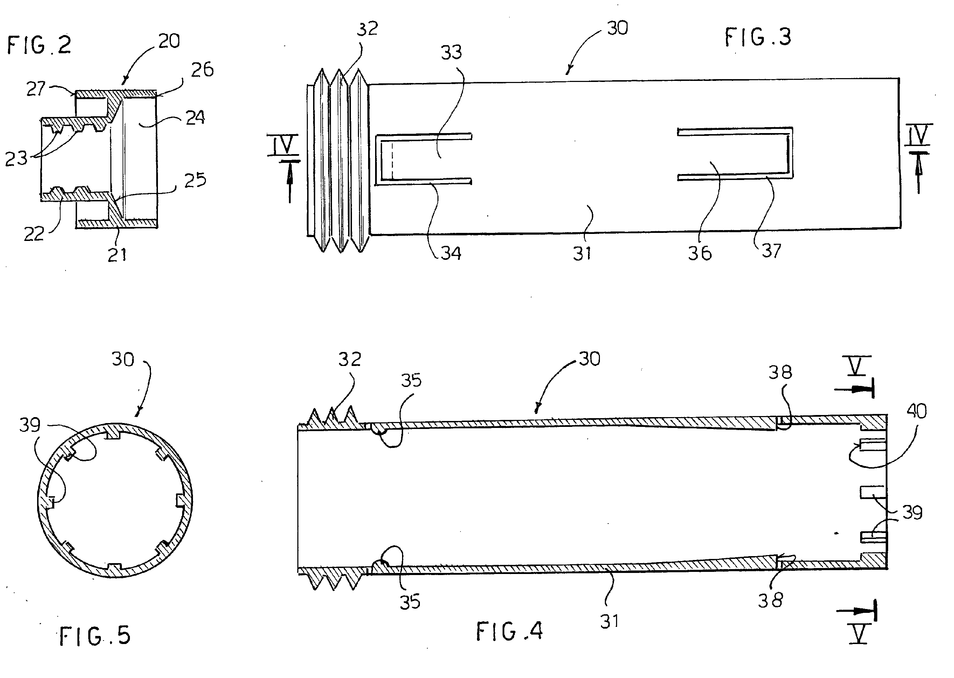

As also shown in Figure 2, the supporting element 20 comprises a body 21, substantially cylindrical and hollow on the inside, having in its front and rear edges respectively a front abutment surface 27 and a rear abutment surface 26. A substantially cylindrical or frusto conical tang 22, connected to the inner wall of the body 21, protrudes axially from the body 21. The cylindrical tang 22 has a smaller diameter than that of the body 21 and has an inner thread 23 able to receive the thread 13 of the needle-carrier 11.

Inside the body 21 of the supporting element 20, starting from the rear part, a first cylindrical seat 24 is defined, having a diameter substantially equal to the outside diameter of the syringe body 1 and a second tapered seat 25 which substantially reproduces the profile of the tapered front end 4 of the syringe body 1. The tapered seat 25 of the supporting element 20 communicates with the inside of the cylindrical threaded tang 22.

As shown also in Figures 3, 4 and 5, the operating element 30 comprises a body 31 substantially in the form of an internally hollow cylindrical sleeve having an inside diameter slightly greater than the outside diameter of the syringe body 1 to be able to slide axially on the syringe body 1 and length substantially equal to the length of the syringe body 1 or in any case greater than the length of the needle 10 to be able to cover the needle 10.

In the front part the operating element 30 has gripping tongues 32, in the form of rings which protrude radially from the outer side surface, to facilitate gripping by the user, avoiding possible sliding of the operating device.

The operating element 30 has a pair of front retaining tongues 33 and a pair of rear stopping tongues 36 disposed longitudinally on the body 31.

The retaining tongues 33 are disposed behind the gripping tongues 32 in diametrically opposite positions. Each retaining tongue 33 is defined by a substantially U-shaped notch 34 formed on the body 31 of the operating element. In this manner the tongues 33 can bend inward and outward with respect to the axis of the operating element 30. As shown in Figure 4, each retaining tongue 33 has at its end a protruding part or ridge 35 which protrudes inward from the inner surface thereof.

In the vicinity of the rear part of the body 31 of the operating element, stopping tongues 36 disposed longitudinally in diametrically opposite positions are provided. Each stopping tongue 36 is defined by a substantially U-shaped notch 37 formed on the body 31 of the operating element. In this manner the stopping tongues 36 can bend inward and outward with respect to the axis of the operating element 30. As shown in Figure 4 each stopping tongue 36 has an inside surface that is tapered so as to define an inwardly protruding abutment surface 38.

At its rear end, the body 30 of the operating element has a plurality of longitudinal ribs 39 which protrude radially inward from the inner side surface of the body 30. Each rib 39 has at its end an abutment surface 40. As shown in Figure 5, the body 31 of the operating element has twelve ribs 39 disposed equidistant from each other, with an angular distance of about 45° from one another.

Assembly and operation of the syringe 100 with its safety accessory 50 will now be described.

The sleeve-type operating element 30 is inserted from its rear onto the syringe body 1 until its rear edge abuts against the flange 3 of the syringe. The supporting element is subsequently mounted on the head of the syringe body so that the tapered part 4 of the syringe body is forcibly wedged into the tapered seat 25 of the supporting element and the head 2 of the syringe body is disposed axially inside the tang 22 of the supporting element leaving a toroidal space between the outer surface of the head 2 of the syringe body and the inner surface of the tang 22 of the supporting element.

The cap 14 is mounted on the needle-carrier 11 so as to cover the needle 10 and thus the needle-carrier 11 supporting the needle 10 and the cap 14 is screwed into the threaded tang 22 of the supporting element 20. As the thread 13 of the needle-carrier screws into the thread 23 of the supporting element, the head 2 of the syringe enters the seat of the needle-carrier 11.

Clearly the needle-carrier 11 can first be screwed onto the supporting element 20 and then the assembly consisting of the supporting element 20 and the needle-carrier 11 is assembled on the head 2 of the syringe body 1. Alternatively, before inserting the sleeve-type operating element 30 on the syringe body 1, the supporting element 20 and needle-carrier 11 assembly can be inserted inside the sleeve element 30 and thus the sleeve element 30 is inserted on the syringe body from the rear and the supporting element 20 is fixed onto the front or head 2 of the syringe body.

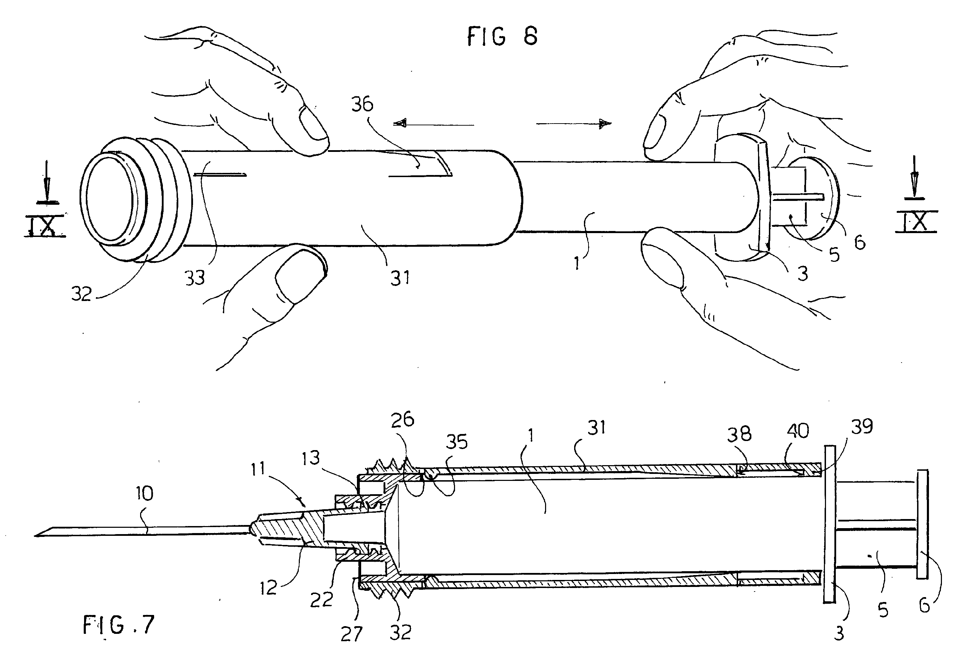

In this situation, as shown in Figures 6 and 7, the syringe is ready for use. The rear edge of the operating element 30 abuts against the flange 3 of the syringe body and the protruding parts 35 of the front tongues of the operating element abut against the rear edge 26 of the supporting element, thus preventing accidental slipping off of the operating element 30. Furthermore, the front edge of the operating element 30 ends in the vicinity of the front edge 27 of the supporting element, thus it does not interfere with the needle 10 of the syringe during injection.

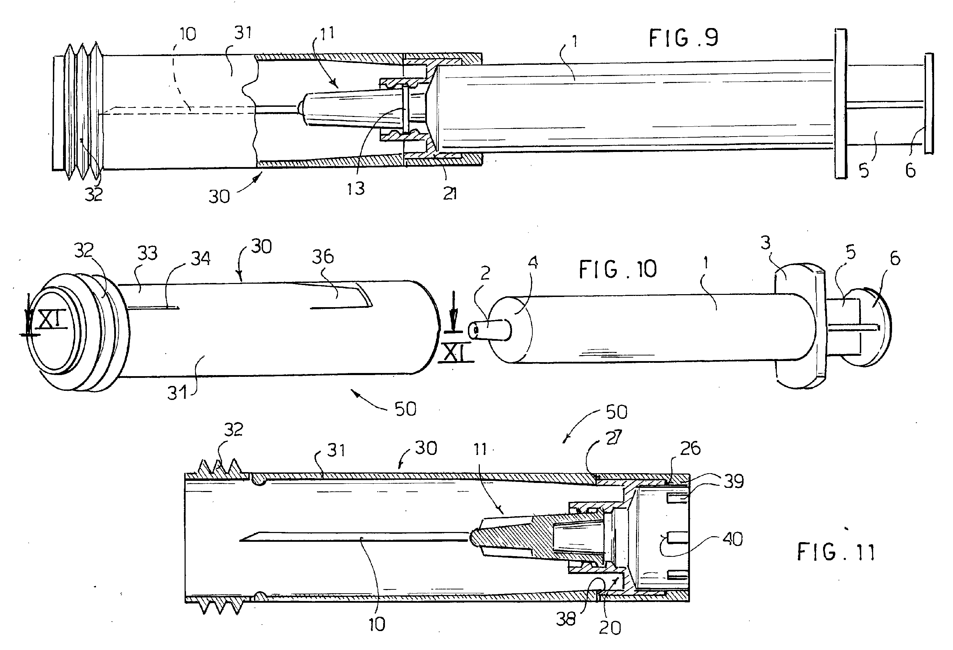

When an injection is to be carried out, the user removes the needle cap 14 and carries out the injection. Once the injection has been carried out, the user slips off the operating element 30 by pulling the operating element 30 and the syringe body 1 in opposite directions as shown by the arrows in Figure 8. During this movement of extraction of the operating element 30, the retaining tongues 33 and the stopping tongues 38 bend outward, respectively when their protruding parts 35 and 38 must go beyond the supporting element 20.

As shown in Figure 9, at the end of the extraction movement of the operating element 30, the abutment surfaces 40 of the ribs 39 of the operating element 30 abut against the rear edge 26 of the supporting element. Consequently a further traction of the operating element 30 causes disengagement and separation of the supporting element 20 of the head 2 of the syringe body.

As shown in Figure 10, as a result, the supporting element 20 with the needle 10 and the needle carrier 11 integral therewith, is in a safe position inside the operating element 30 which, having a greater length than the length of the needle 10, acts as a protection against accidental needle sticks. In this situation the operating element 30 with the supporting element 20 therein can be sent for disposal without any risk of accidental needle sticks.

Furthermore, it should be noted that in this situation the supporting element 20 cannot be removed from the operating element; in fact, as shown in Figure 11, the front edge 27 of the supporting element 20 abuts against the abutment surface 38 of the stopping tongues 36 of the operating element and the rear edge 26 of the supporting element abuts against the abutment surface 40 of the ribs 39 of the operating element.

Obviously, the syringe equipped with the safety accessory according to the invention can be used indifferently to carry out injections or to take samples.

Numerous changes and modifications of detail within the reach of a person skilled in the art can be made to the present invention without thereby departing from the scope of the invention as set forth in the appended claims.

## Claims

1. A safety accessory for a hypodermic syringe, the hypodermic syringe (100) comprising:
- a syringe body (1) hollow on the inside and open at the front and rear,
- a plunger sliding inside the syringe body (1) with an injection stroke extending from a retracted syringe-filling position to a forward syringe-emptying position, said plunger being provided at the rear with a shaft (5) that can be operated manually and brought out of the syringe body through the rear end thereof, and
- an injection needle (10) integral with a needle-carrier (11) attachable to the front end (2) of the syringe body (1),
**characterized in that** said safety accessory (50) comprises:
- a supporting element (20) removably mounted on the front part of said syringe body (1) and able to receive said needle-carrier (11), and
- an operating element (30) slidably mounted on said syringe body (1) and comprising abutment means (39, 40) able to cooperate with said supporting element (20) during sliding thereof for extraction from the syringe body (1), to cause disengagement of the supporting element (20) from the front part of said syringe body, so that said supporting element (20) integral with the needle-carrier (11) is retained inside said operating element (30).

2. A safety element (50) according to claim 1, **characterized in that** said operating element (30) is shaped as a cylindrical sleeve, hollow on the inside, having an inside diameter slightly greater than the outside diameter of said syringe body (1) and a length slightly greater than the length of said needle (10).

3. A safety accessory (50) according to claim 1 or 2, **characterized in that** said supporting element (20) has an internally hollow substantially cylindrical body (21), with an inside diameter substantially equal to the outside diameter of said syringe body, said cylindrical body (21) defining a front abutment edge (27) and a rear abutment edge (26).

4. A safety accessory (50) according to claim 3, **characterized in that** said abutment means of said operating element (30) are ribs (39) disposed in the inner surface of the rear part of said operating element (30) so as to define abutment surfaces (40) able to abut against the rear edge (26) of said supporting element (20).

5. A safety accessory (50) according to any one of the preceding claims, **characterized in that** said operating element (30) comprises stopping means (36) able to lock said supporting element inside said operating element (30) after said abutment means (39, 40) have stopped sliding of the operating element (30) with consequent disengagement of said supporting element (20) from the syringe body (1).

6. A safety accessory (50) according to claim 5, **characterized in that** said stopping means (36) of said operating element (30) comprise flexible tongues (36) defined by a substantially U-shaped notch on said body of said operating element and having an inwardly protruding abutment surface (38) able to abut against the front abutment surface (27) of said supporting element (20).

7. A safety accessory (50) according to any one of the preceding claims, **characterized in that** said operating element (30) comprises retaining means (33) such as to allow retention of said operating element (30) on the syringe body (1).

8. A safety accessory (50) according to claim 7, **characterized in that** said retaining means (33) of said operating element (30) comprise flexible tongues (33) defined by a substantially U-shaped notch on said body of said operating element and having an inwardly protruding abutment surface (35) such as to abut on the rear abutment surface (26) of said supporting element (20), when said supporting element is mounted on the front of said syringe body.

9. A safety accessory (50) according to any one of the preceding claims, **characterized in that** said operating element (30) comprises gripping means (32) such as to facilitate gripping of said operating element by the user.

10. A safety accessory (50) according to claim 9, **characterized in that** said gripping means (32) of said operating element (30) comprise annular tongues (32) disposed on the outer surface of the body of said operating element.

11. A safety accessory (50) according to any one of the preceding claims, **characterized in that** said supporting element (20) comprises a cylindrical tang (22) having an inner thread (23) able to receive an outer thread (13) provided in said needle-carrier (11).

12. A safety syringe, **characterized in that** it comprises a safety accessory according to any one of the preceding claims.
